# EUROPÄISCHE PATENTSCHRIFT

(11) **EP 2 819 744 B1**
(45) Veröffentlichungstag und Bekanntmachung des Hinweises auf die Patenterteilung: **20.04.2016**
(21) Anmeldenummer: 13707814.3
(22) Anmeldetag: 26.02.2013
(51) Int. Cl.: A61N 1/36, A61N 1/04

(54) **ELEKTRODENANORDNUNG**
ELECTRODE ARRANGEMENT
DISPOSITIF À ÉLECTRODE

(30) Priorität: 02.03.2012 DE 102012004021; 25.07.2012 DE 102012014764
(43) Veröffentlichungstag der Anmeldung: 07.01.2015
(73) Patentinhaber: Cerbomed GmbH, 91052 Erlangen (DE)
(72) Erfinder: HARTLEP, Andreas, 83607 Holzkirchen (DE); BECK, Christoph, 79115 Freiburg (DE); BÄR, Stefan, 90556 Cadolzburg (DE)
(74) Vertreter: Gosdin, Michael
(86) Internationale Anmeldenummer: PCT/EP2013/000545
(87) Internationale Veröffentlichungsnummer: WO 2013/127512

(56) Entgegenhaltungen:
- DE-A1-102005 003 735
- DE-A1-102010 015 277
- DE-A1-102010 015 278
- DE-A1-102010 022 030
- US-A- 5 514 175
- US-A1- 2008 021 517
- US-A1- 2008 249 594
- US-A1- 2011 160 811
- US-A1- 2011 166 624

## Beschreibung

Die Erfindung betrifft eine Elektrodenanordnung zur Aufbringung eines transkutanen elektrischen Stimulationsreizes auf die Oberfläche eines Abschnittes des menschlichen Ohrs, die ein am oder im Ohr anbringbares Halteelement sowie mindestens eine Elektrode aufweist, die in oder an einem Elektrodenträger angeordnet ist, wobei das Halteelement eine Linearführung aufweist, in der eine Haltestange in Richtung einer Längsachse des Halteelements linear verschieblich angeordnet ist, wobei an der Haltestange der Elektrodenträger angeordnet ist, wobei der Elektrodenträger mindestens einen Trägerabschnitt aufweist, auf dem die Elektrode angeordnet ist, wobei die Elektrode mindestens einen zylindrischen und/oder kugelabschnittsförmigen und/oder kegelförmigen Oberflächenabschnitt aufweist.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zu invasiven als auch zu non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Elektrodenanordnung der eingangs genannten Art ist aus der DE 10 2010 054 165 B3 bekannt. Eine ähnliche Elektrodenanordnung zeigt die EP 2 026 872 B1**.** Hier ist eine Elektrodenanordnung beschrieben, die ein vollständig in der Pinna (Ohrmuschel) unterbringbares Gehäuse umfasst. Von diesem aus erstrecken sich zwei gebogene, drahtförmige Abschnitte, wobei diese als federelastische Halterungen ausgebildet sind. Damit kann die Elektrodenanordnung durch sanftes Einklemmen in der Pinna in die benötigte Position gebracht werden, so dass der Gehörgang mit einem transkutanen Stimulationsreiz beaufschlagt werden kann. Andere Stimulationsvorrichtungen offenbaren die DE 10 2005 003 735 A1**,** die US 5 514 175 und die DE 10 2010 015 277 A1**.**

Wenngleich die vorbekannte Elektrodenanordnung bereits zu guten Behandlungsergebnissen führt, haben sich in der Praxis gewisse Nachteile der Anordnung herausgestellt.

Da naturbedingt die Elektrodenanordnungen mit ihren Elektroden sehr klein bauen müssen, ist die mechanische Stabilität der benötigten Verbindungen, insbesondere der Elektroden mit ihren Anschlusskabeln, bisher nicht optimal gelöst. Daher kann es zu einer ungenügenden Halterung der Elektroden kommen, was dadurch begünstigt wird, dass ein sehr weiches anschmiegsames Material (insbesondere Silikon) für die Halterung eingesetzt werden muss, um einen hinreichenden Tragekomfort zu erzielen. Mitunter kommt es bei entsprechender mechanischer Belastung der Elektrodenanordnung bei ihrem Einsetzen in den Gehörgang zu irreparablen Schäden an den Kontakten der Elektroden.

Des weiteren wurde es als nachteilig erkannt, dass es schwer ist, eine bestehende Elektrodenanordnung gegebenenfalls auf die individuelle Größe der Pinna bzw. generell des Ohrs anzupassen. Dies führt mitunter zu hohem Aufwand beim Anlegen der Elektrodenanordnung bzw. zu einem nicht optimalen Tragekomfort.

Nachteilig ist es weiterhin bei manchen vorbekannten Elektrodenanordnungen, dass das Hörvermögen negativ beeinflusst werden kann.

Des weiteren ist die Kontaktqualität der Elektroden mitunter noch nicht optimal, da die von den Elektroden akquirierten Hautareale nicht ausreichend groß sind. Dies ergibt sich bei manchen vorbekannten Lösungen durch die Art und Weise, wie die Elektroden an einem Trägerelement angeordnet sind.

Weiterhin ist es wünschenswert, trotz Standardisierung der Stimulationsvorrichtungen Maßnahmen vorzusehen, um in einfacher und kostengünstiger Weise eine Anpassung der Stimulationsvorrichtung an ein individuelles Ohr zu ermöglichen.

Der Erfindung liegt daher die **Aufgabe** zugrunde, eine Elektrodenanordnung der gattungsgemäßen Art so fortzubilden, dass die genannten Nachteile überwunden werden. Es soll also eine Elektrodenanordnung vorgeschlagen werden, mit der das Anlegen der Elektrodenanordnung in einfacher und bequemer Weise möglich ist. Das Hörvermögen soll dabei beim Tragen der Elektrodenanordnung so wenig wie möglich negativ beeinflusst werden. Die Kontaktqualität der Elektroden soll durch optimale Ausgestaltung verbessert werden.

Die **Lösung** dieser Aufgabe durch die Erfindung ist dadurch gekennzeichnet, dass zwei Trägerabschnitte und/oder Achselemente zum Halten einer Elektrode vorgesehen sind, die unter einem Winkel angeordnet sind, der zwischen 140° und 180° liegt.

Dabei ist bevorzugt ein zylindrischer und ein kugelabschnittsförmiger Oberflächenabschnitt oder ein kegelförmiger und ein kugelabschnittsförmiger Oberflächenabschnitt vorgesehen.

Eine bevorzugte Lösung sieht vor, dass die Elektrode in ihrem dem Trägerabschnitt zugewandten Bereich zylindrisch ausgebildet ist, wobei sich an den zylindrischen Bereich der Elektrode in deren vom Trägerabschnitt abgewandten Endbereich ein kugelabschnittsförmiger Bereich anschießt. Bei dieser Lösung kann ferner vorgesehen werden, dass der Übergang der Elektrode von ihrem zylindrischen Bereich zum Trägerabschnitt durch einen kegelförmigen Bereich gebildet wird, wobei der zylindrische Bereich einen größeren Durchmesser aufweist als derjenige des Trägerabschnitts.

Der Elektrodenträger kann dabei einen von der Haltestange kommenden Halteabschnitt aufweisen, an dessen Ende mindestens zwei in verschiedene Richtungen weisende Trägerabschnitte mit jeweiligen Elektroden angeordnet sind.

Der mindestens eine Trägerabschnitt kann ein Achselement aufweisen, auf dem die Elektrode aufgebracht, insbesondere aufgesteckt, ist. Die Elektrode kann dabei auf dem Achselement stoffschlüssig, formschlüssig und/oder reibschlüssig befestigt sein.

Eine alternative Lösung sieht vor, dass der mindestens eine Trägerabschnitt, vorzugsweise zusammen mit dem Halteabschnitt, durch einen Spritzgießprozess ausgebildet ist, wobei die Elektrode durch den Spritzgießprozess in situ mit dem Trägerabschnitt verbunden ist.

Der Halteabschnitt kann eine Längsachse aufweisen, die zur Längsachse des Halteelements einen Winkel einschließt, der zwischen 20° und 80°, vorzugsweise zwischen 40° und 70°, beträgt.

Eine bevorzugte Ausgestaltung der Erfindung sieht vor, dass die beiden Trägerabschnitte und/oder Achselemente unter einem Winkel zwischen 165° und 175° angeordnet sind.

Weiterhin kann vorgesehen werden, dass das Halteelement ein Auflageteil umfasst, das zur Auflage im Cavum conchae des Ohrs ausgebildet und vorgesehen ist.

Der Elektrodenträger ist zumeist an einem axialen Ende der Haltestange angeordnet.

Der Elektrodenträger weist, insbesondere als Elektrodenkopf ausgebildet, bevorzugt mindestens eine Stimulationselektrode und mindestens eine Referenzelektrode auf.

Die Linearführung des Halteelements kann durch eine Ausnehmung gebildet werden, die in einem Schnitt senkrecht zur Längsachse entlang der Längsachse eine gleichbleibende Form, insbesondere eine Kreisform, aufweist; die Haltestange weist dann bevorzugt zumindest über einen Teil ihrer Erstreckung einen kreisförmigen Querschnitt auf.

Ferner können additiv oder alternativ auch Feststellmittel vorgesehen werden, die die relative lineare Position zwischen dem Halteelement und der Haltestange blockieren, d. h. die Linearverschiebung mittels der Linearführung verhindern.

Weiterhin können Federmittel zwischen der Linearführung und der Haltestange wirksam angeordnet sein, so dass die Haltestange relativ zur Linearführung in Richtung der Längsachse elastisch vorspannbar ist.

Generell kann vorgesehen werden, dass durch entsprechende Maßnahmen eine gewünschte Elastizität des Elektrodenträgers relativ zum Halteelement erreicht wird. Die genannte Integration eines Federelements ist hier nur eine Möglichkeit. Es kann durch geeignete konstruktive Maßnahmen gleichermaßen erreicht werden, dass die Elastizität bzw. Federkonstante in einem gewünschten Bereich liegt. So ist es beispielsweise möglich, einen Abschnitt der Haltestange durch eine metallische Blattfeder zu verstärken und gleichzeitig das Material der Haltestange in diesem Bereich zurückzunehmen oder ganz hierauf zu verzichten. Die Blattfeder ermöglicht eine Federelastizität in eine definierte Richtung, während die Verbindung zwischen dem Halteelement und dem Elektrodenträger in anderen Belastungsrichtungen steifer bleibt.

Aus dem oben Gesagten wird verständlich, dass der Begriff der "Haltestange" hier weit zu interpretieren ist und sich keinesfalls auf klassische stangenförmige Strukturen beschränkt.

Die Haltestange kann in ihrem axialen Endbereich, in dem der Elektrodenträger angeordnet ist, weiterhin mindestens eine seitliche Einkerbung oder mindestens einen seitlichen Einschnitt aufweisen, um im axialen Endbereich die Biegesteifigkeit der Haltestange um eine zur Längsachse senkrechte Richtung zu reduzieren. Die Anpassung des Elektrodenträgers an die Oberflächentopographie der zu stimulierenden Haut wird dadurch erleichtert.

Der ringförmige Abschnitt des Auflageteils kann an mindestens einer Umfangsstelle eine Unterbrechung aufweisen. Damit kann erreicht werden, dass eine vereinfachte Anpassung des kreisringförmigen Abschnitts an den Auflagebereich im Ohr bei verschiedenen Ohrgrößen gegeben ist.

Die Teile der Elektrodenanordnung bestehen bevorzugt - soweit Hautkontakt gegeben ist - aus einem weichen Material, wobei speziell an ein Elastomermaterial gedacht ist, insbesondere an Silikon oder an ein Material, das Silikon aufweist. Indes bestehen der Elektrodenkopf und die Haltestange bevorzugt aus einem thermoplastischen oder duroplastischen Kunststoffmaterial, beispielsweise auch aus Polyurethan.

Es sei erwähnt, dass die vorgeschlagene Elektrodenanordnung auch nur eine Elektrode aufweisen kann. Möglich ist, dass eine separate Referenzelektrode eingesetzt wird, die außerhalb der Elektrodenanordnung (z. B. hinter dem Ohr) platziert wird und mit dem Stimulationsgerät in elektrischer Verbindung steht. Möglich ist es auch, dass zwar - wie im Ausführungsbeispiel - zwei oder mehr Elektroden vorhanden sind, dass allerdings eine weitere Referenzelektrode außerhalb der Elektrodenanordnung zum Einsatz kommt.

Eine weitere Fortbildung sieht vor, dass die Elektrodenanordnung, vorzugsweise im Bereich des Auflageteils bzw. dessen ringförmigen Abschnitts mit einem akustischen Geber (Lautsprecher) versehen ist. Damit wird es möglich, auch akustische Signale während der Elektrostimulation dem Benutzer der Elektrodenanordnung zuzuleiten, was auch zu Unterhaltungszwecken geschehen kann.

Vorteilhaft ist es, dass durch die vorgeschlagene Ausgestaltung der Elektrodenanordnung eine recht stabile Konstruktion erreicht wird, ohne den Tragekomfort negativ zu beeinflussen. Die Elektroden werden stabil und zuverlässig in der benötigten Position gehalten, da sie über die Haltestange am relativ stabilen Halteelement angeordnet sind.

Durch die zweiteilige Bauweise ist es im Übrigen möglich, die Haltestange samt Elektrodenkopf auf der einen Seite mit dem Halteelement auf der anderen Seite zu kombinieren. Dies eröffnet die Möglichkeit, unterschiedlich große Elemente miteinander zu kombinieren. Eine bevorzugte Lieferausstattung sieht daher mehr als eine Haltestange samt Elektrodenkopf und/oder mehr als ein Halteelement vor. Der Anwender kann dann die Elemente mit der für seine Verhältnisse optimalen Größe auswählen und zusammenfügen, um eine optimal passende Elektrodenanordnung zur Verfügung zu haben.

Insbesondere die Halteelemente können so in einfacher Weise an verschieden große Ohren bzw. unterschiedliche Ohrformen angepasst werden. Der Elektrodenkopf bzw. die Elektroden liegen dabei stets mit einem definierten Druck auf der Haut auf.

In der Zeichnung sind Ausführungsbeispiele der Erfindung dargestellt. Es zeigen:
- Fig. 1: in perspektiver Ansicht eine Elektrodenanordnung gemäß der Erfindung,
- Fig. 2: in perspektivischer Ansicht die Elektrodenanordnung nach Fig. 1 von vorne betrachtet,
- Fig. 3: in perspektivischer Ansicht die Elektrodenanordnung nach Fig. 1, von unten her gesehen,
- Fig. 4: die Draufsicht auf den vorderen Abschnitt der Elektrodenanordnung nach Fig. 1,
- Fig. 5: in perspektivischer Ansicht die Elektrodenanordnung nach Fig. 1 von vorne gesehen,
- Fig. 6: in perspektiver Ansicht den vorderen Abschnitt der Elektrodenanordnung nach Fig. 1, wobei die Elektroden selber demontiert sind,
- Fig. 7: in perspektivischer Ansicht den vorderen Abschnitt der Elektrodenanordnung nach Fig. 1,
- Fig. 8: die Seitenansicht der vorderen Hälfte der Elektrodenanordnung nach Fig. 1 mit einer ersten Ausgestaltung eines ringförmigen Kontaktelements,
- Fig. 9: die Seitenansicht der vorderen Hälfte der Elektrodenanordnung nach Fig. 1 mit einer zweiten Ausgestaltung eines ringförmigen Kontaktelements,
- Fig. 10: die Seitenansicht der vorderen Hälfte der Elektrodenanordnung nach Fig. 1 mit einer dritten Ausgestaltung eines ringförmigen Kontaktelements,
- Fig. 11: in perspektiver Ansicht die Elektrodenanordnung nach Fig. 1,
- Fig. 12: in perspektiver Ansicht den vorderen Teil der Elektrodenanordnung nach Fig. 1, gesehen aus einer anderen Richtung,
- Fig. 13: die Ansicht eines Ohrs mit einer Elektrodenanordnung, die in das Ohr eingesetzt ist,
- Fig. 14: die Darstellung nach Fig. 13, aus einer anderen Richtung gesehen,
- Fig. 15: die Seitenansicht der Elektrodenanordnung nach Fig. 1,
- Fig. 16a: einen teilweise geschnitten dargestellten Teil eines Auflageteils der Elektrodenanordnung, mit einem ringförmigen Kontaktelement gemäß einer ersten Ausführungsform,
- Fig. 16b: einen geschnitten dargestellten Teil des ringförmigen Kontaktelements gemäß einer zweiten Ausführungsform,
- Fig. 16c: einen geschnitten dargestellten Teil des ringförmigen Kontaktelements gemäß einer dritten Ausführungsform und
- Fig. 17: schematisch in der Seitenansicht Rastmittel, die zwischen einem Halteelement und einer Haltestange der Elektrodenanordnung wirksam angeordnet sind.

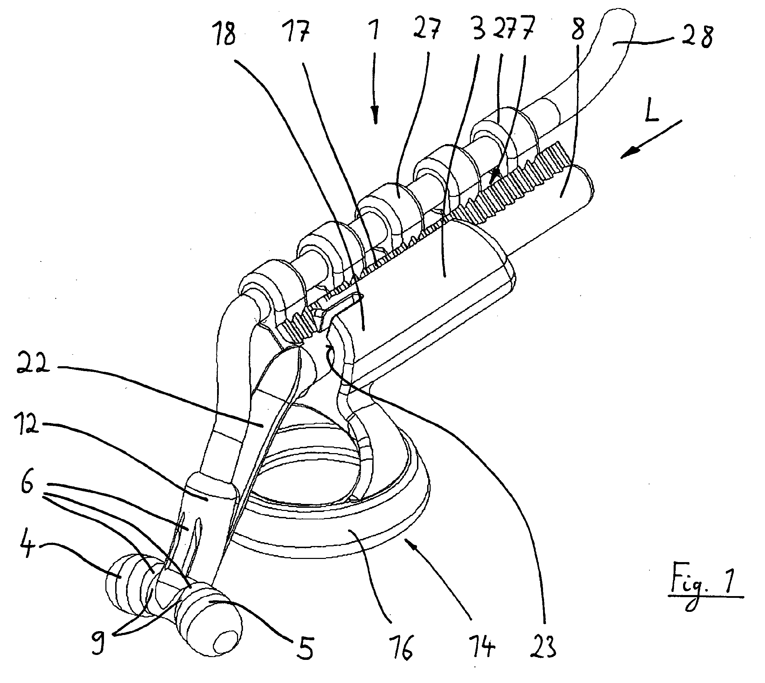

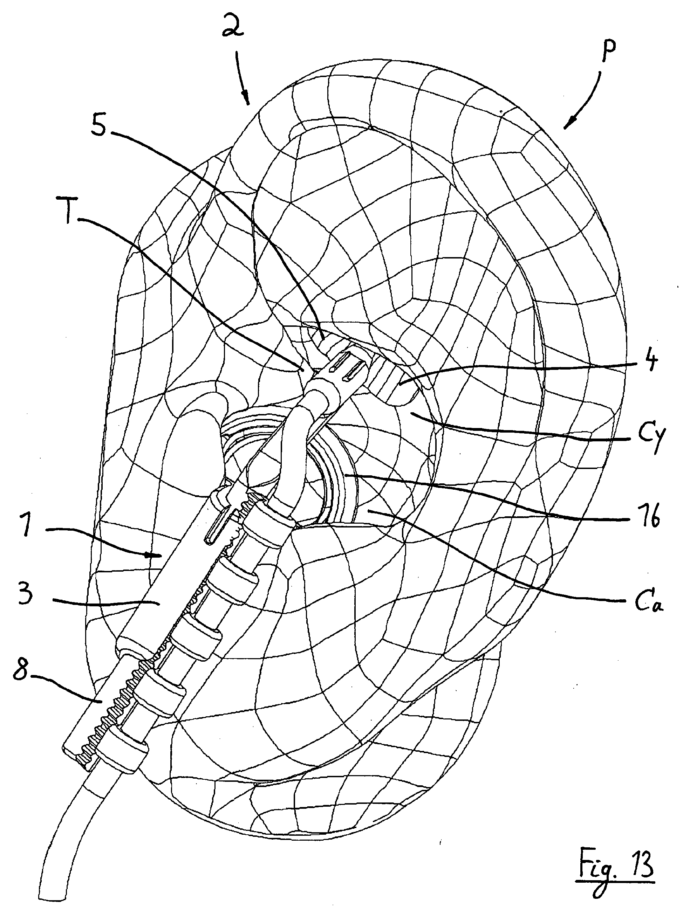

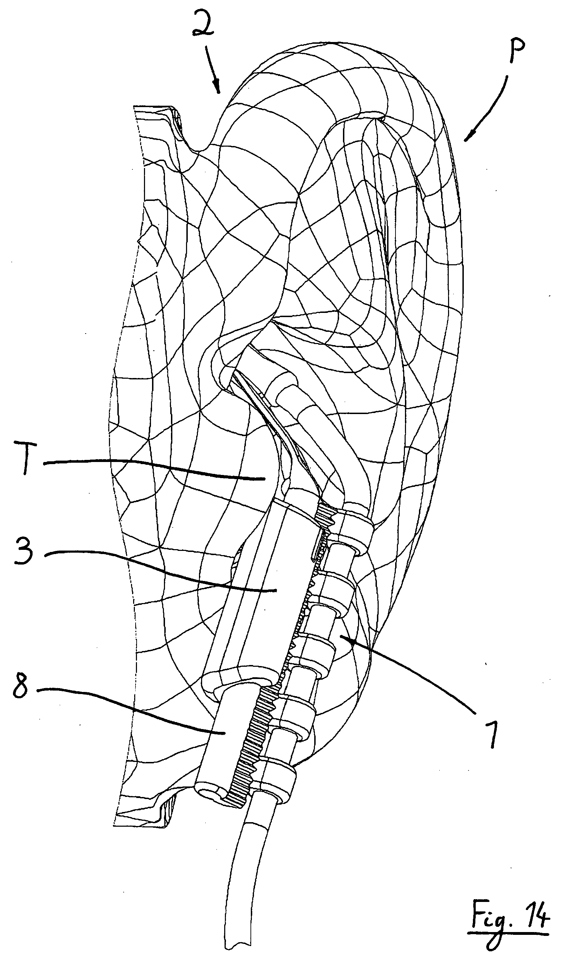

In Fig. 1 ist eine Elektrodenanordnung 1 in Form einer Otoplastik dargestellt, die in ein in Fig. 13 bzw. Fig. 14 dargestelltes Ohr 2 eines Menschen eingesetzt werden kann, um eine transkutane Elektrostimulation der Hautoberfläche im Bereich des Ohres vornehmen zu können.

Mit der Elektrodenanordnung 1 kann konkret auf einen Oberflächenbereich des Ohres eine transkutane elektrische Nervenstimulation vorgenommen werden. Hierfür weist die Elektrodenanordnung eine Stimulationselektrode und eine Referenzelektrode (s. unten) auf, zwischen denen ein elektrisches Potential erzeugt wird; die hierfür nötigen Mittel sind im Stand der Technik hinlänglich bekannt, so dass sie hier nicht weiter beschrieben werden müssen. Exemplarisch wird auf die DE 10 2005 003 735 B4 der Anmelderin verwiesen und hierauf ausdrücklich Bezug genommen.

Die Elektrodenanordnung 1 hat als wesentliche Baubestandteile ein Halteelement 3 sowie eine Haltestange 8. Die Haltestange 8 trägt an einem axialen Ende über ein Verbindungselement 22 einen Elektrodenträger 6, der mit zwei Elektroden 4, 5 versehen ist, nämlich mit einer Stimulationselektrode 4 und einer (baugleichen) Referenzelektrode 5. Der Elektrodenträger 6 hält die beiden Elektroden 4, 5 auf einem gewünschten Abstand und ist zwischen den Elektroden 4, 5 als Brückenelement ausgebildet. Das Halteelement 3 hat einen zentralen Abschnitt, der von einer Linearführung 7 dominiert wird, diese wird aus Abschnitten der Teile 3 und 8 gebildet. Hierbei handelt es sich um einen sich in eine Längsrichtung L erstreckenden Materialabschnitt mit stabartiger Form, in den eine kreisförmige Ausnehmung 23 eingeformt ist.

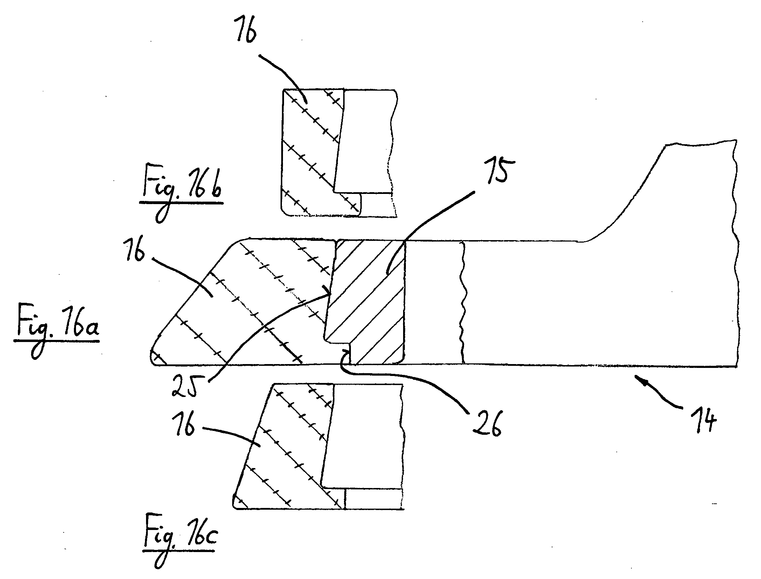

Am vorderen Ende des Halteelements 3 ist ein Auflageteil 14 angeformt. Das Auflageteil 14 weist einen ringförmigen Abschnitt 15 (s. insbesondere Fig. 16) auf. Der ringförmige Abschnitt 15 ist von einem ringförmigen Kontaktelement 16 umgeben, das aus weichem Kunststoffmaterial besteht und auf der Hautoberfläche aufliegt, wie sich aus Fig. 13 ergibt.

Wesentlich ist dabei, dass das Halteelement 3 mittels der Linearführung 7 in der Lage ist, die Haltestange 8 in Richtung der Längsachse L linear zu verschieben.

Damit wird es möglich, den Abstand zwischen dem Elektrodenträger 6 und somit der Elektroden 4, 5 und insbesondere dem Auflageteil 14 zu verändern und auf ein gewünschtes Maß einzustellen.

Diese Einstellmöglichkeit wird genutzt, um die Elektrodenanordnung nach Platzierung im Ohr 2 so zu verstellen, dass sie bei gutem Tragekomfort elastischen Halt im Ohr 2 findet.

Hierzu wird auf Fig. 13 und Fig. 14 verwiesen. Hier ist zu sehen, dass die Elektrodenanordnung 1 ins Ohr 2 eingesetzt und durch lineare Verschiebung der Haltestange 8 relativ zum Halteelement 3 so eingestellt wurde, dass sich die Elektrodenanordnung 1 elastisch aufgrund der Topographie des Ohrs 2 festgespannt hat. Konkret wurde die Elektrodenanordnung 1 so in der Pinna P des Ohrs 2 angeordnet, dass der Elektrodenträger 6 im Bereich der Cymba conchae Cy zu liegen kommt, während der Auflageteil 14 mit seinem ringförmigen Kontaktelement 16 im Bereich des Cavum conchae Ca aufliegt.

Demgemäß kommt das ringförmige Kontaktelement 16 unterhalb des Tragus T zu liegen.

Infolge einer (materialbedingten) Federelastizität der Haltestange 8 bzw. wegen eines bei Bedarf in die Haltestange 8 integrierten Federelements (z. B. Federdrahts) spannt sich somit die Elektrodenanordnung 1 nach entsprechender linearer Einstellung der Haltestange 8 relativ zum Halteelement 3 elastisch vor, so dass ein hinreichender Halt im Ohr gegeben ist. Wie in Fig. 13 zu sehen ist, erlaubt die ringförmige Ausgestaltung des ringförmigen Kontaktelements 14 ein weitgehend ungehindertes Hörempfinden.

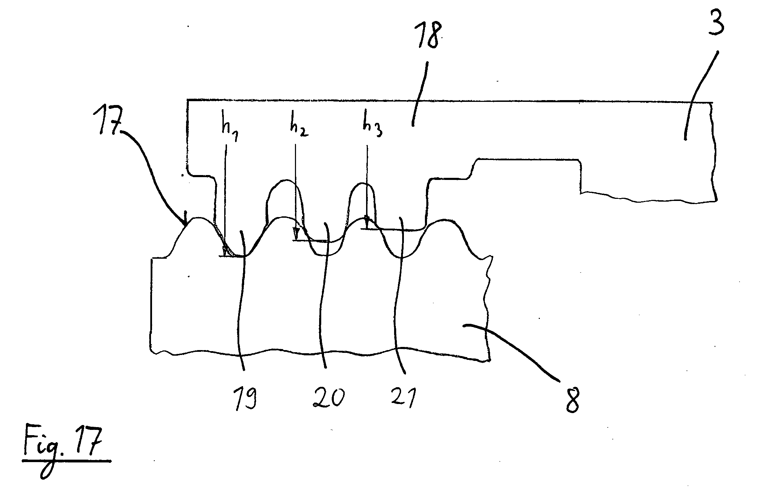

Wie weiter in Fig. 1 gesehen werden kann, ist die Haltestange 8 aufgrund ihrer Ausformung relativ zum Halteelement 3 nicht um die Längsachse L verdrehbar. Dies wird durch eine zahnstangenförmige Kontur 17 erreicht, die an der Haltestange 8 angeformt ist und durch eine sich radial von der kreisförmigen Ausnehmung 23 des Halteelements 3 aus erstreckende Ausnehmung tritt. Ein Abschnitt des Halteelements 3 ist zudem als Eingriffselement 18 ausgebildet (s. hierzu Fig. 17), wobei durch die zahnstangenförmige Kontur 17 einerseits und das Eingriffselement 18 andererseits Rastmittel gebildet werden, mittels denen die Haltestange 8 relativ zum Halteelement 3 ratschenartig, d. h. schrittweise linear in Richtung der Längsachse L verschoben werden kann.

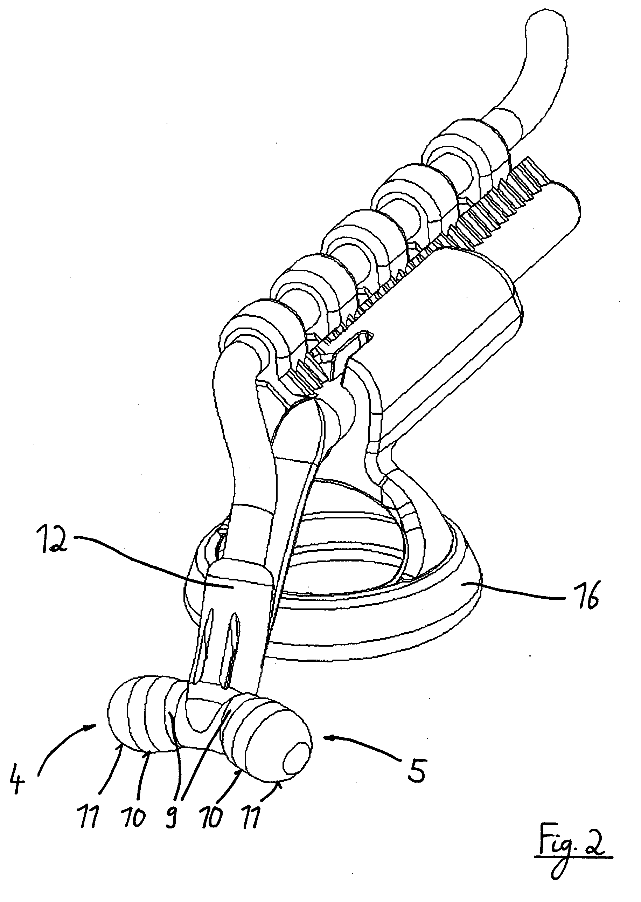

Ein wesentlicher Aspekt der vorliegenden Erfindung ist die Ausgestaltung der Elektroden 4, 5 am Elektrodenträger 6. Wie es am besten in Fig. 2 gesehen werden kann, haben die Elektroden 5, 6 eine Ausformung, die sich aus einem zylindrischen Abschnitt 10 und einem kugelabschnittförmigem Abschnitt 11 zusammensetzt. Auch kegelförmige Abschnitte können additiv oder alternativ vorgesehen werden. Damit wird erreicht, dass die Elektroden - abhängig vom Andruck - relativ große Hautflächenareale akquirieren, d. h. eine große Auflagefläche auf der Haut haben, was die transkutane Stimulation entsprechend begünstigt. Die Elektroden 4, 5 sind hiernach jedenfalls abschnittsweise ellipsoid oder perlenförmig ausgebildet. In vorteilhafter Weise ergibt sich eine verbesserte Anpassung der Elektroden an die gegebene Anatomie des Ohrs.

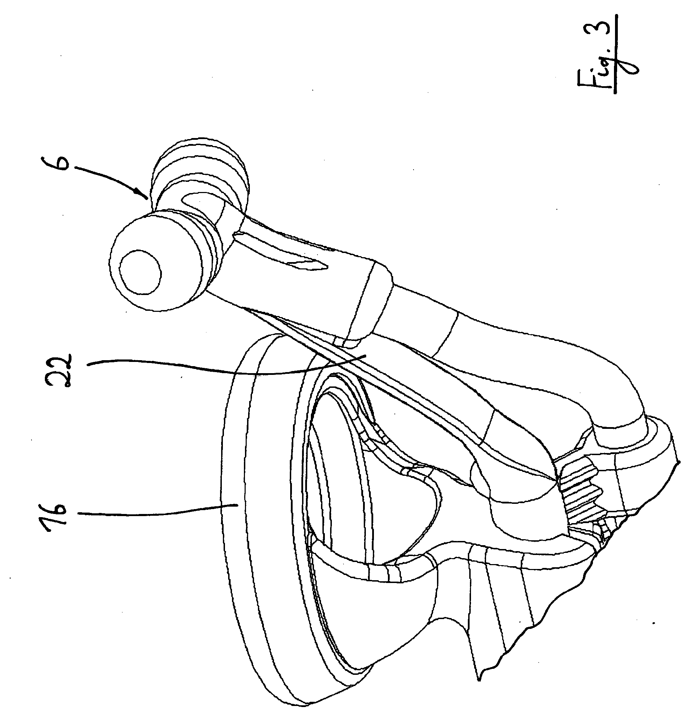

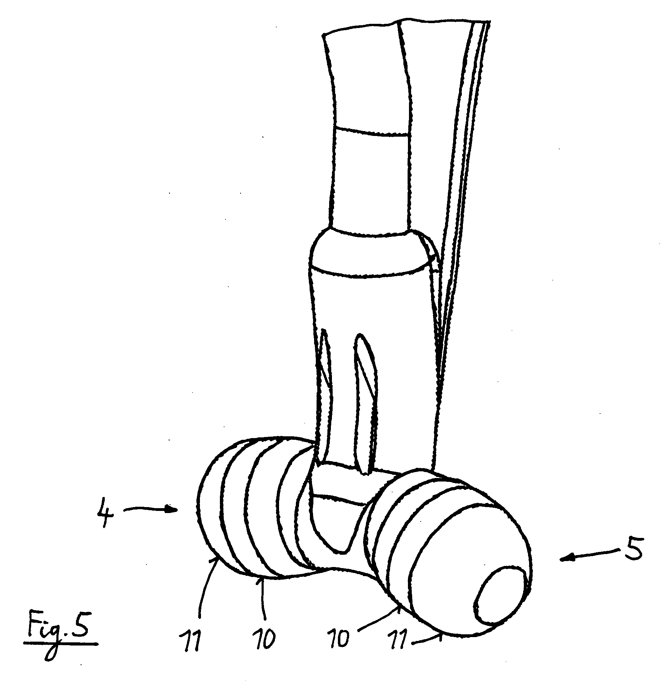

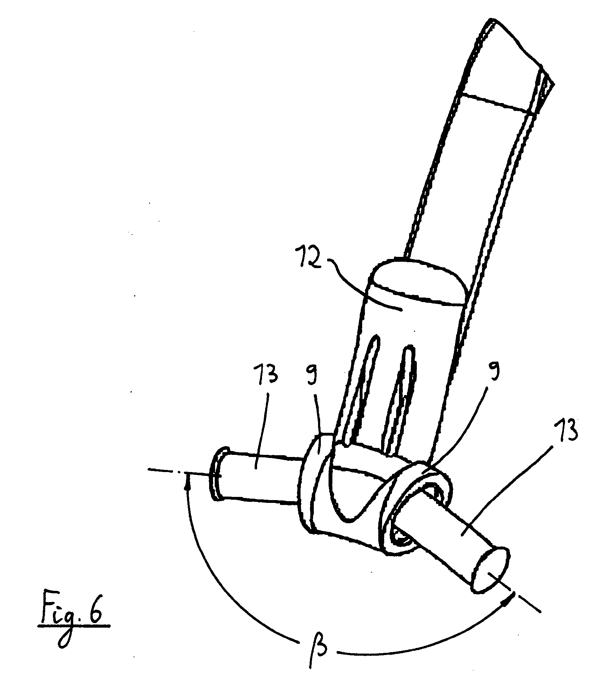

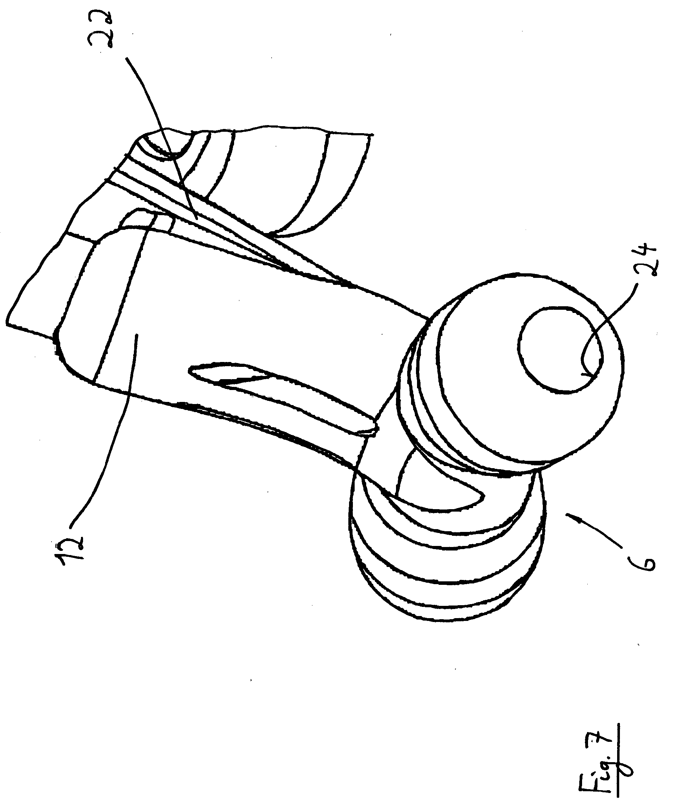

Eine mögliche Anbringung der Elektroden 4, 5 am Elektrodenträger 6 geht aus den Figuren 3 bis 7 hervor. Hiernach umfasst der Elektrodenträger 6 einen Halteabschnitt 12, an dem zwei Trägerabschnitte 9 angeordnet sind. Jeder Trägerabschnitt 9 hat - s. hierzu Fig. 6 - ein Achselement 13, auf das je eine Elektrode 4, 5 aufgesteckt werden kann. Hierzu weist die Elektrode 4, 5 eine korrespondierende Bohrung 24 auf (s. Fig. 7), so dass sich nach Aufstecken der Elektroden 4, 5 auf die Achselemente 13 gemäß Fig. 6 die Anordnung ergibt, wie sie in den Figuren 3, 4, 5 und 7 zu erkennen ist.

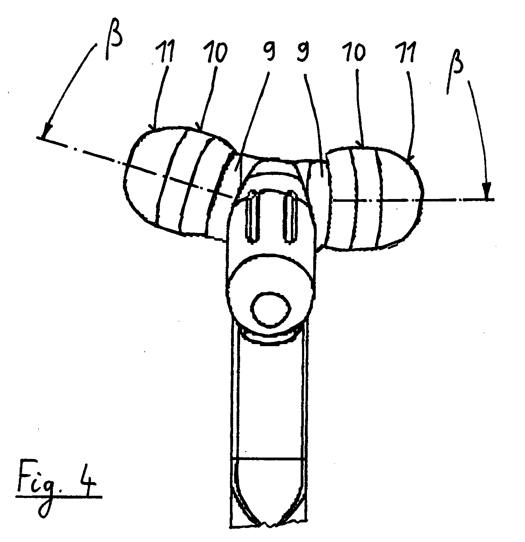

In diesem Zusammenhang sei angemerkt, dass die beiden z. B. in Fig. 4, 5 und 6 zu sehenden Trägerabschnitte 9 bzw. Achselemente 13 unter einem sich zur Haut hin öffnenden Winkel knapp unter 180° angeordnet sind - im Ausführungsbeispiel sind es ca. 170° -, so dass eine optimale Anlage der Elektroden auf der Haut sichergestellt ist. Besagter Winkel ist in den Figuren 4 und 6 mit β angegeben.

Die Elektroden 4, 5 können dabei beispielsweise auf den Achselementen 13 festgeklebt sein.

Eine alternative Lösung sieht vor, dass die Elektroden 4, 5 beim Spritzgießen des Elektrodenträgers 6 samt Trägerabschnitten 9 mit angespritzt werden. Die Elektroden sind daher dann in günstiger Weise nicht mehr umspritzt, sondern auf einer Kunststoffseele angeordnet. Die Anordnung der Elektroden ist damit optimal zum vorgesehenen Zweck positioniert.

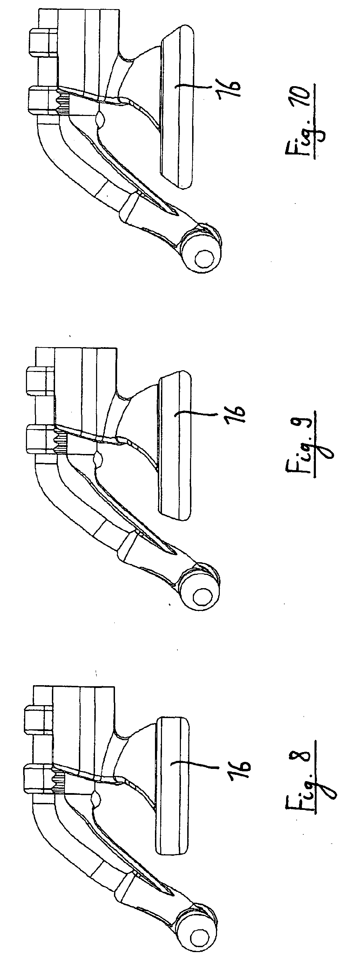

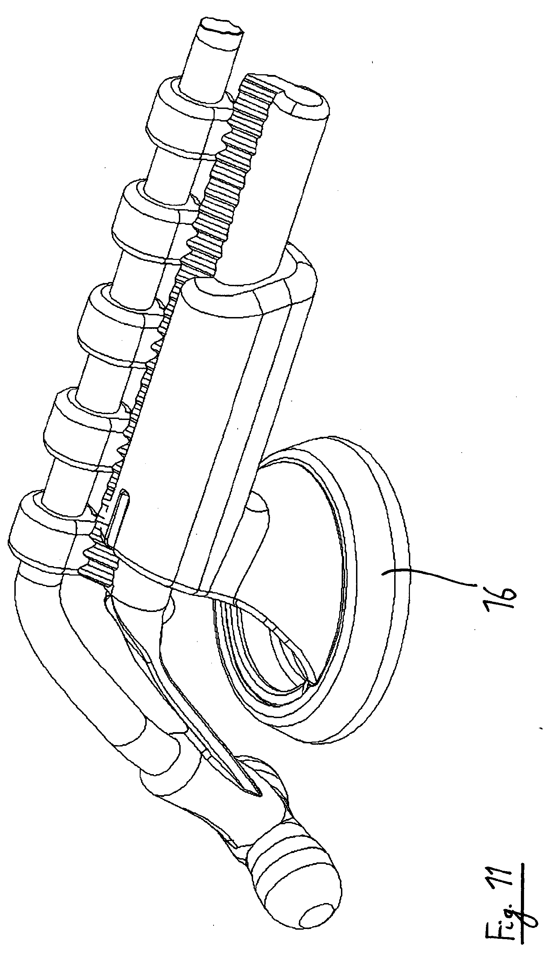

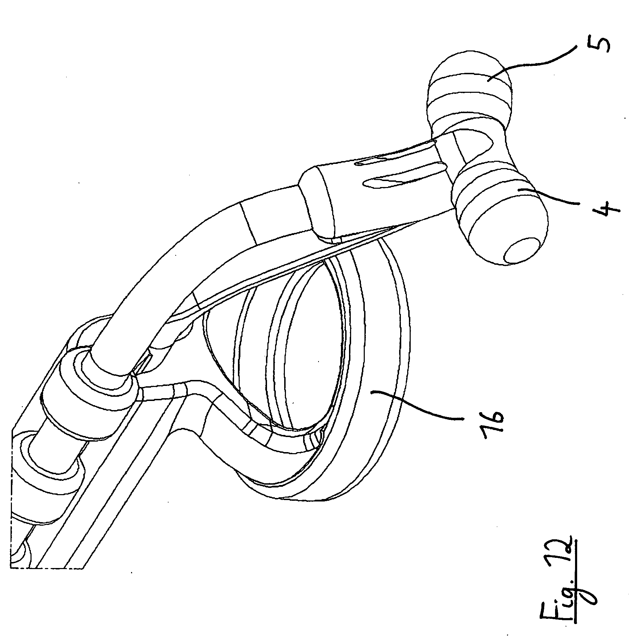

Aus der Zusammenschau der Figuren 8 bis 12 und 16 ergibt sich eine weitere sehr vorteilhafte Ausgestaltung der Erfindung: Das Auflageteil 14 besteht aus einem ringförmigen Abschnitt 15, das von einem lösbar aufgesetzten ringförmigen Kontaktelement 16 umgeben wird. Für das aus weichem Material bestehende Kontaktelement 16 sind dabei verschiedene Formen möglich, wie sie einmal aus den Figuren 8, 9 und 10 zu erkennen sind und analog auch aus den Figuren 16a, 16b und 16c, wo die jeweiligen Schnitte dargestellt sind.

Die Ausformung des ringförmigen Abschnitts 15 ist kongruent zur Form des ringförmigen Kontaktelements 16. Vorgesehen ist eine leicht konische Ausformung 25 des radial außenliegenden Bereichs des ringförmigen Abschnitts 15, ferner ist eine Ausnehmung 26 im ringförmigen Abschnitt 15 vorgesehen. Das ringförmige Kontaktelement 16 ist hierzu kongruent ausgebildet, so dass es mit Hinterschnitt auf den ringförmigen Abschnitt 15 aufgeschoben werden kann und somit hier hinreichenden Halt findet.

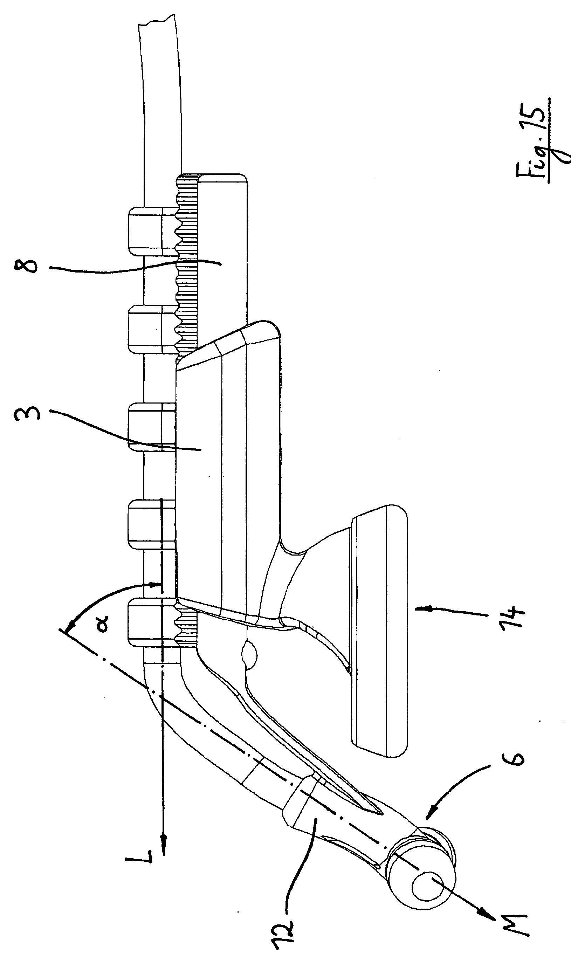

In Fig. 15 ist zu erkennen, dass der Halteabschnitt 12 des Elektrodenträgers 6 eine Längsachse M aufweist, die die Längsachse L unter einem Winkel α schneidet, der im Ausführungsbeispiel bei ca. 65° liegt. Damit sind die Elektroden in einem spitzen Winkel zur Auflagefläche angeordnet.

Wie in Fig. 17 weiter gesehen werden kann, bildet das Eingriffselement 18 einen freigestellten Abschnitt des Halteelements 3, der über Einschnitte ins Material des Halteelements freigestellt und somit elastisch gehalten ist. An der der zahnstangenförmigen Kontur 17 zugewandten Seite sind am Eingriffselement 18 drei Zähne 19, 20 und 21 angeformt. Diese sind hinsichtlich ihrer Höhe gestaffelt ausgeführt (s. die Höhen h₁, h₂ und h₃), so dass die Rastfunktion primär vom Zahn 19 und dann nur noch unterstützend von den Zähnen 20 und 21 bestimmt wird.

Am radial außenliegenden Ende des Abschnitts mit der zahnstangenförmigen Kontur 17 der Haltestange 8 sind mehrere Haltemittel 27 in Form von ringförmigen Strukturen angeformt, die dazu dienen, ein Kabel 28 (bevorzugt mit 3-adriger Kevlarlitze und von biokompatiblem Mantelmaterial umgeben), das die Elektroden 4, 5 mit Strom versorgt, zugentlastet zu halten. Die Haltemittel 27 dienen also als Kabelführungsösen, in denen das Kabel 28 geführt und zugentlastet wird. Das Kabel 28 kann dabei beim Spritzgießen der einstückig ausgeführten Teile 8, 17 und 27 mit umspritzt werden. Bei der Herstellung des Elektrodenträgers 6 durch Spritzgießen kann in analoger Weise das Kabel 28 mit umspritzt werden, um eine hermetische Versiegelung aller Nähte zu erreichen.

An die Haltestange 8 schließt sich - als Verbindungsabschnitt zum Elektrodenträger 6 - ein Verbindungselement 22 an, das die Funktion hat, den Elektrodenträger 6 von der Höhe, in der sich die Linearführung 7 befindet, zur zu stimulierenden Hautoberfläche hinunter zu führen. Wie bereits erläutert, muss dies so erfolgen, dass der Elektrodenträger 6 elastisch gegen die Hautoberfläche drückt und bevorzugt im Übrigen auch eine Vorspannung in Richtung der Längsachse L erzeugt.

Hierfür wird auf ein Material für das Verbindungselement 22 zurückgegriffen, die die gewünschte Elastizität aufweist; dieses Element hat also bevorzugt federelastische Eigenschaften. Möglich ist es auch, dass das Verbindungselement 22 ein Federelement zu integrieren. Hierbei kann es sich beispielsweise um einen Federdraht handeln, der beim Spritzgießen der Haltestange 8 samt Verbindungselement 22 mit umspritzt wird. Wie beispielsweise aus Fig. 1 zu erkennen ist, ist das Verbindungselement 22 im Querschnitt nicht kreisförmig, sondern abgeflacht ausgebildet. Hiermit erhöht sich die Elastizität des Elektrodenträgers 6 in eine Richtung normal zur Längsachse L.

Dabei erfolgen die Werkstoffwahl, gegebenenfalls die Integration eines Federelements und die geometrische Ausformung der Haltestange fachmännisch derart, dass eine gewünschte Elastizität vorliegt.

### Bezugszeichenliste:

- 1: Elektrodenanordnung
- 2: Ohr
- 3: Halteelement
- 4: Elektrode (Stimulationselektrode)
- 5: Elektrode (Referenzelektrode)
- 6: Elektrodenträger
- 7: Linearführung
- 8: Haltestange
- 9: Trägerabschnitt
- 10: zylindrischer Abschnitt der Elektrode
- 11: kugelabschnittsförmiger Abschnitt der Elektrode
- 12: Halteabschnitt
- 13: Achselement
- 14: Auflageteil
- 15: ringförmiger Abschnitt
- 16: ringförmiges Kontaktelement
- 17, 18: Rastmittel
- 17: zahnstangenförmige Kontur / als Wellenbahn ausgebildete Kontur
- 18: Eingriffselement
- 19: Zahn
- 20: Zahn
- 21: Zahn
- 22: Verbindungselement
- 23: kreisförmige Ausnehmung
- 24: Bohrung
- 25: konische Ausformung
- 26: Ausnehmung
- 27: Haltemittel
- 28: Kabel

- L: Längsachse
- M: Längsachse des Halteabschnitts

- α: Winkel
- β: Winkel

- Ca: Cavum conchae
- Cy: Cymba conchae
- T: Tragus
- P: Pinna

## Patentansprüche

1. Elektrodenanordnung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie mindestens eine Elektrode (4, 5) aufweist, die in oder an einem Elektrodenträger (6) angeordnet ist, wobei das Halteelement (3) eine Linearführung (7) aufweist, in der eine Haltestange (8) in Richtung einer Längsachse (L) des Halteelements (3) linear verschieblich angeordnet ist, wobei an der Haltestange (8) der Elektrodenträger (6) angeordnet ist, wobei der Elektrodenträger (6) mindestens einen Trägerabschnitt (9) aufweist, auf dem die Elektrode (4, 5) angeordnet ist, wobei die Elektrode (4, 5) mindestens einen zylindrischen (10) und/oder kugelabschnittsförmigen (11) und/oder kegelförmigen Oberflächenabschnitt aufweist,
**dadurch gekennzeichnet,**
**dass** zwei Trägerabschnitte (9) und/oder Achselemente (13) zum Halten einer Elektrode (4, 5) vorgesehen sind, die unter einem Winkel (β) angeordnet sind, der zwischen 140° und 180° liegt.

2. Elektrodenanordnung nach Anspruch 1, **dadurch gekennzeichnet, dass** die Elektrode (4, 5) in ihrem dem Trägerabschnitt (9) zugewandten Bereich zylindrisch (10) ausgebildet ist, wobei sich an den zylindrischen Bereich der Elektrode (4, 5) in deren vom Trägerabschnitt (9) abgewandten Endbereich ein kugelabschnittsförmiger (11) Bereich anschießt.

3. Elektrodenanordnung nach Anspruch 2, **dadurch gekennzeichnet, dass** der Übergang der Elektrode (4, 5) von ihrem zylindrischen Bereich (10) zum Trägerabschnitt (9) durch einen kegelförmigen Bereich gebildet wird, wobei der zylindrische Bereich (10) einen größeren Durchmesser aufweist als derjenige des Trägerabschnitts (9).

4. Elektrodenanordnung nach einem der Ansprüche 1 bis 3, **dadurch gekennzeichnet, dass** der Elektrodenträger (6) einen von der Haltestange (8) kommenden Halteabschnitt (12) aufweist, an dessen Ende mindestens zwei in verschiedene Richtungen weisende Trägerabschnitte (9) mit jeweiligen Elektroden (4, 5) angeordnet sind.

5. Elektrodenanordnung nach einem der Ansprüche 1 bis 4, **dadurch gekennzeichnet, dass** der mindestens eine Trägerabschnitt (9) ein Achselement (13) aufweist, auf dem die Elektrode (4, 5) aufgebracht, insbesondere aufgesteckt, ist.

6. Elektrodenanordnung nach Anspruch 5, **dadurch gekennzeichnet, dass** die Elektrode (4, 5) auf dem Achselement (13) stoffschlüssig, formschlüssig und/oder reibschlüssig befestigt ist.

7. Elektrodenanordnung nach einem der Ansprüche 1 bis 4, **dadurch gekennzeichnet, dass** der mindestens eine Trägerabschnitt (9), vorzugsweise zusammen mit dem Halteabschnitt (12), durch einen Spritzgießprozess ausgebildet ist, wobei die Elektrode (4, 5) durch den Spritzgießprozess in situ mit dem Trägerabschnitt (9) verbunden ist.

8. Elektrodenanordnung nach einem der Ansprüche 1 bis 7, **dadurch gekennzeichnet, dass** der Halteabschnitt (12) eine Längsachse (M) aufweist, die zur Längsachse (L) des Halteelements (3) einen Winkel (α) einschließt, der zwischen 20° und 80°, vorzugsweise zwischen 40° und 70°, beträgt.

9. Elektrodenanordnung nach einem der Ansprüche 1 bis 8, **dadurch gekennzeichnet, dass** die beiden Trägerabschnitte (9) und/oder Achselemente (13) unter einem Winkel (β) zwischen 165° und 175° angeordnet sind.

10. Elektrodenanordnung nach einem der Ansprüche 1 bis 9, **dadurch gekennzeichnet, dass** das Halteelement (3) ein Auflageteil (14) umfasst, das zur Auflage im Cavum conchae (Ca) des Ohrs (2) ausgebildet und vorgesehen ist.

## Claims

1. Electrode arrangement (1) for applying of a transcutaneous electrical stimulation stimulus onto the surface of a section of the human ear (2), which comprises a holding element (3) to be attached at or in the ear (2) as well as at least one electrode (4, 5), which electrode is arranged in or at an electrode carrier (6), wherein the holding element (3) comprises a linear guide (7) in which a supporting rod (8) is arranged linear movable in the direction of a longitudinal axis (L) of the holding element (3), wherein the electrode carrier (6) is arranged at the supporting rod (8), wherein the electrode carrier (6) comprises at least one carrier section (9) on which the electrode (4, 5) is arranged, wherein the electrode (4, 5) comprises at least one cylindrical (10) and/or spherical segment shaped (11) and/or conical surface section,
**characterized in that**
two carrier sections (9) and/or axle elements (13) are provided for holding of an electrode (4, 5), which are arranged below an angle (β), which is between 140° and 180°.

2. Electrode arrangement according to claim 1, **characterized in that** the electrode (4, 5) is designed cylindrical (10) in its section facing the carrier section (9), wherein a spherical segment shaped (11) section adjoins to the cylindrical region of the electrode (4, 5) in its end remote from the carrier section (9).

3. Electrode arrangement according to claim 2, **characterized in that** the transition of the electrode (4, 5) from its cylindrical region (10) to the carrier section (9) is formed by a conical region, wherein the cylindrical region (10) has a bigger diameter than that one of the carrier section (9).

4. Electrode arrangement according to one of claims 1 to 3, **characterized in that** the electrode carrier (6) comprises a holding section (12) which comes from the supporting rod (8), on which end at least two carrier sections (9) pointing into different directions are arranged with the respective electrodes (4, 5).

5. Electrode arrangement according to one of claims 1 to 4, **characterized in that** the at least one carrier sections (9) comprises an axle element (13) on which the electrode (4, 5) is applied, particularly is plugged on.

6. Electrode arrangement according to claim 5, **characterized in that** the electrode (4, 5) on the axle element (13) is attached adhesive bonded, form-fitted and/or is fictionally engaged.

7. Electrode arrangement according to one of claims 1 to 4, **characterized in that** the at least one carrier sections (9), preferably together with the holding section (12), is formed by an injection molding process, wherein the electrode (4, 5) is connected with the carrier section (9) in situ by the injection moulding process.

8. Electrode arrangement according to one of claims 1 to 7, **characterized in that** the holding section (12) comprises a longitudinal axis (M), which encloses an angle (α) to the longitudinal axis (L) of the holding section (3), which is between 20° and 80°, preferably between 40° and 70°.

9. Electrode arrangement according to one of claims 1 to 8, **characterized in that** the two carrier sections (9) and/or axle elements (13) are provided below an angle (β), which is between 165° and 175°.

10. Electrode arrangement according to one of claims 1 to 9, **characterized in that** the holding element (3) comprises a resting part (14) that is designed and provided for resting in the Cavum conchae (Ca) of the ear (2).

## Revendications

1. Dispositif à électrode (1) destiné à appliquer une stimulation électrique transcutanée à la surface d'une partie de l'oreille humaine (2), lequel dispositif comporte un élément de maintien (3) qui peut être placé sur ou dans l'oreille (2) et au moins une électrode (4, 5) qui est disposée dans ou sur un support d'électrode (6), dans lequel l'élément de maintien (3) comporte un guide linéaire (7) dans lequel une tige de maintien (8) est disposée de manière à pouvoir effectuer un déplacement linéaire dans la direction d'un axe longitudinal (L) de l'élément de maintien (3), dans lequel le support d'électrode (6) est disposé sur la tige de maintien (8), dans lequel le support d'électrode (6) comporte au moins une partie de support (9) sur laquelle est disposée l'électrode (4, 5), dans lequel l'électrode (4, 5) comporte au moins une partie de surface cylindrique (10) et/ou en forme de partie de sphère (11) et/ou de forme conique,
**caractérisé**
**en ce qu'**il est prévu pour maintenir une électrode (4, 5) deux parties de support (9) et/ou deux éléments d'axe (13) qui sont disposés sous un angle (β) compris entre 140° et 180°.

2. Dispositif à électrode selon la revendication 1, **caractérisé en ce que** l'électrode (4, 5) est réalisée de manière cylindrique (10) dans sa région qui est tournée vers la partie de support (9), dans lequel une région en forme de partie de sphère (11) se raccorde à la région cylindrique de l'électrode (4, 5) dans sa région d'extrémité qui est tournée à l'opposé de la partie de support (9).

3. Dispositif à électrode selon la revendication 2, **caractérisé en ce que** la transition de l'électrode (4, 5) de sa région cylindrique (10) à la partie de support (9) est formée par une région de forme conique, dans lequel la région cylindrique (10) présente un diamètre supérieur à celui de la partie de support (9).

4. Dispositif à électrode selon l'une quelconque des revendications 1 à 3, **caractérisé en ce que** le support d'électrode (6) comprend une partie de maintien (12) partant de la tige de maintien (8), à l'extrémité de laquelle sont disposées au moins deux parties de support (9) orientées dans des directions différentes et comportant des électrodes respectives (4, 5).

5. Dispositif à électrode selon l'une quelconque des revendications 1 à 4, **caractérisé en ce que** l'au moins une partie de support (9) comprend un élément d'axe (13) sur lequel est montée, notamment enfichée, l'électrode (4, 5).

6. Dispositif à électrode selon la revendication 5, **caractérisé en ce que** l'électrode (4, 5) est fixée à l'élément d'axe (13) par continuité de matériau, par complémentarité de forme et/ou par frottement.

7. Dispositif à électrode selon l'une quelconque des revendications 1 à 4, **caractérisé en ce que** l'au moins une partie de support (9) est réalisée par un processus de moulage par injection, de préférence en association avec la partie de maintien (12), dans lequel l'électrode (4, 5) est reliée in situ à la partie de support (9) par le processus de moulage par injection.

8. Dispositif à électrode selon l'une quelconque des revendications 1 à 7, **caractérisé en ce que** la partie de maintien (12) présente un axe longitudinal (M) qui inclut un angle (α) par rapport à l'axe longitudinal (L) de l'élément de maintien (3), compris entre 20° et 80°, de préférence, entre 40° et 70°.

9. Dispositif à électrode selon l'une quelconque des revendications 1 à 8, **caractérisé en ce que** les deux parties de support (9) et/ou les deux éléments d'axe (13) sont disposés sous un angle (β) compris entre 165° et 175°.

10. Dispositif à électrode selon l'une quelconque des revendications 1 à 9, **caractérisé en ce que** l'élément de maintien (3) comprend une pièce d'appui (14) qui est réalisée et prévue pour un appui dans la cavité de cornet (Ca) de l'oreille (2).
